(19) Europäisches Patentamt
European Patent Office
Office européen des brevets

(11) Veröffentlichungsnummer: **0 063 323**
**B1**

# (12) EUROPÄISCHE PATENTSCHRIFT

(45) Veröffentlichungstag der Patentschrift: **30.10.85**

(51) Int. Cl.⁴: **C 07 D307/935**, A 61 K 31/34// C07C149/40

(21) Anmeldenummer: **82103025.1**

(22) Anmeldetag: **08.04.82**

(54) **5-Substituierte 4-Oxo-PGI1-Derivate, Verfahren zu deren Herstellung sowie diese Verbindungen enthaltende pharmazeutische Präparate.**

(30) Priorität: **14.04.81 HU 96581**

(43) Veröffentlichungstag der Anmeldung: **27.10.82 Patentblatt 82/43**

(45) Bekanntmachung des Hinweises auf die Patenterteilung: **30.10.85 Patentblatt 85/44**

(84) Benannte Vertragsstaaten: **BE CH DE FR GB IT LI NL SE**

(56) Entgegenhaltungen: **US-A- 4 258 199**

**Die Akte enthält technische Angaben, die nach dem Eingang der Anmeldung eingereicht wurden und die nicht in dieser Patentschrift enthalten sind.**

(73) Patentinhaber: **CHINOIN Gyogyszer és Vegyészeti Termékek Gyára RT.
To utca 1-5
H-1045 Budapest IV (HU)**

(72) Erfinder: **Simonidesz, Vilmos, Dr. Dipl.Ing.Chem.
Fenyö u.13
H-1016 Budapest (HU)**
Erfinder: **Ivanics, József, Dipl.Ing.Chem.
Elem u.20
H-1045 Budapest (HU)**
Erfinder: **Galambos, Géza, Dipl.Ing.Chem.
Lavotta u. 60
H-1104 Budapest (HU)**
Erfinder: **Papp geb.Behr, Agnes, Dipl.Ing.Chem.
Deák F.u. 33
H-1041 Budapest (HU)**
Erfinder: **Kovács, Gábor, Dr. Dipl.Ing.Chem.
Róna park 4
H-1142 Budapest (HU)**
Erfinder: **Skopál, Judit, Dipl.Ing.Chem.
Szakasits A. u.34/b
H-1115 Budapest (HU)**
Erfinder: **Szilágyi, Ildiko, Dipl.-Ing. Chem.
Bajcsy Zs. u.26
H-2364 Ocsa (HU)**

(74) Vertreter: **Lotterhos, Hans Walter, Dr.-Ing.
Lichtensteinstrasse 3
D-6000 Frankfurt am Main 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 063 323 B1

Jouve, 18, rue St-Denis, 75001 Paris, France

**Beschreibung**

Die Erfindung betrifft neue, biologisch aktive, optisch aktive oder racemische 5-substituierte $PGI_1$-Derivate der allgemeinen Formel I

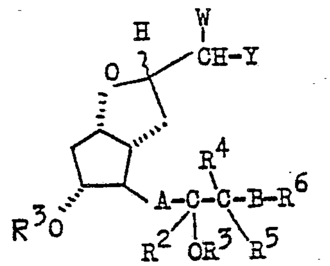

(I)

sowie deren mit pharmakologisch verträglichen Kationen gebildete Salze, worin

W $-COOR^1$, $-CN$, $-NO_2$, $C_{1-4}$-Alkanoyl, $C_{1-4}$-Alkyl—S—, Phenyl-S—, $C_{1-4}$-Alkyl-phenyl—S—, $C_{1-4}$-Alkyl—$SO_2$—, Phenyl-$SO_2$- oder $C_{1-4}$-Alkyl-phenyl-$SO_2$—,

$R^1$ Wasserstoff, $C_{1-6}$-Alkyl, $C_{3-10}$-Cycloalkyl, Benzyl oder Phenyl,

Y Wasserstoff (wenn W $NO_2$ ist), $-COOR^1$, $-COR^1$, $-CN$ oder $-\underset{\underset{Z}{\|}}{C}-X-Q$ bedeutet, worin

Z zwei Wasserstoff (wenn W $NO_2$ ist) oder Sauerstoff,

X $-CH_2-CH_2-$, $-CH=CH-$, $-CH_2-C(CH_3)_2-$, $-(CH_3)_2C-CH_2-$, Phenyl-S—CH—$CH_2$—, —$CH_2$—CH—S—Phenyl oder Cyclopropylen,

Q $-COOH$, $-COOR^1$, $-CH_2OH$, $-COL^1$,

M ein pharmakologisch verträgliches Kation und

$L^1$ $-NH_2$, $-NHR^1$ oder $-NR^1R^1$ ist,

A trans-CH=CH—, —C≡C— oder $-CH_2-CH_2-$,

$R^2$ Wasserstoff der α- oder β-Konfiguration, Methyl oder Äthyl,

$R^3$ Wasserstoff, $C_{1-4}$-Alkanoyl, gegebenenfalls substituiertes Aroyl- oder eine Tri-$C_{1-4}$-Alkyl-silyl-Schutzgruppe,

$R^4$ und $R^5$ unabhängig voneinander Wasserstoff oder $C_{1-4}$-Alkyl,

$R^6$ gerades oder verzweigtes $C_{1-6}$-Alkyl, Phenyl, das durch $C_{1-4}$-Alkyl, Halogen oder durch Halogen substituiertes $C_{1-4}$-Alkyl, substituiert sein kann, oder substituiertes Heteroaryl und

B Methylen, Sauerstoff oder —NH bedeuten.

Die neuen 5-substituierten $PGI_1$-Derivate der allgemeinen Formel I sind als Prostacyclinanaloga zu bezeichnen, die — ebenso wie das $PGI_2$ — z. B. die Blutplättchenaggregation hemmen und den Blutdruck senken. Außerdem sind sie Synthesezwischenprodukte für die Herstellung von neuen, stabilen $PGI_2$-Analoga, von 4-Oxo-$PGI_2$ und 4-Oxo-$PGI_1$ sowie deren Derivaten. Die Verbindungen der allgemeinen Formel I hemmen in einer Konzentration von 1-100 γ/ml die durch ADP oder Arachidonsäure oder Kollagen ausgelöste Blutplättchenaggregation, sie wirken auf den Kreislauf, hemmen die Sekretion von Magensäure, wirken auf das gastrointestinale System und weisen zahlreiche pharmakologische Eigenschaften auf.

a. Gefäßerweiternde Wirkung

Diese neuen Prostacyclinanaloga der Erfindung üben eine ebenso starke periphere gefäßerweiternde Wirkung aus wie das Prostacyclin. So können sie zur Vorbeugung und Behandlung von unterschiedlichen Erkrankungen des Gefäßsystems, z. B. bei Arteriosklerose obliterans, Myocardinfarkt, Thrombose, Gefäßverengung und hohem Blutdruck, eingesetzt werden. Ihre Verabreichung kann in Form einer intravenösen Infusion, intramuskulär oder subkutan, in Form von Tabletten oder Kapseln erfolgen. Die tägliche Dosis beträgt 0,1-100 mg je nach Alter und Schwere der Krankheit.

Einige Verbindungen, insbesondere 5-Nitro-$PGI_1$ und dessen Derivate weisen starke Selektivität auf, neben stark gefäßerweiternder Wirkung verfügen sie über geringere sonstige prostacyclinartige Wirkung.

Ihre hämodynamische Wirkungskraft ist genauso groß wie die Wirkung von $PGI_2$ (die mit $PGI_2$ äquipotente Dosis beträgt 0,1-0,5 μg/kg Körpergewicht bei narkotisierten Katzen), während ihre Antiaggregationswirkung das 1/1 000-1/10 000 der von $PGI_2$ beträgt. Die bei Humanblut mit $1 \times 10^{-6}$ Mol/ml ADP ausgelöste Blutplättchenaggregation wurde nach der Born-Methode gemessen in einer Konzentration von 5-100 μg/ml ($IC_{50}$) gehemmt.

b. Hemmung der Blutplättchenaggregation

Diese neuen Prostacyclinanaloga hemmen die Blutplättchenaggregation, vermindern das Haften der Blutplättchen aneinander, verhindern die Bildung von Thrombosen, üben eine Desaggregationswirkung auf, d. h. sie lösen entstandene Thrombosen auf. So sind sie zur Behandlung und zur Vorbeugung von

Thrombosen, zur Behandlung von Anginaerkrankungen, bei speziellen Herzoperationen zur Beseitigung des Blutgerinnens beim Prozeß des extracorporalen Kreislaufs und zur Sicherung von optimalen Parametern geeignet.

Vorteilhaft werden sie oral in Form von Tabletten oder Kapseln täglich 2-4 mal angewendet. Die tägliche Dosis kann zwischen 0,05-100 mg schwanken.

In vitro hemmen sie in einer Konzentration von 0,1-100 $\gamma$-ml die durch Arachidonsäure, ADP und Kollagen ausgelöste Blutplättchenaggregation.

c. Hemmung der Magensäuresekretion

Die neuen Prostacyclinanaloga der Erfindung sind in der Human- und der Tierheilkunde zum Hemmen und Beeinflussen der Magensäuresekretion geeignet, sie senken oder verhindern die Möglichkeit der Geschwürbildung und beschleunigen die Heilung schon entstandener Geschwüre. Ihre Dosis beträgt täglich 0,01-10 mg/kg Körpergewicht in Form einer intravenösen oder subkutanen Injektion oder Infusion. Bei oraler Anwendung beträgt die Dosis täglich 1-100 mg.

d. Bronchienerweiterung

Diese neuen Prostacyclinanaloga sind zur Behandlung von Asthma als Bronchienerweiterer und Inhibitoren der Mediatorstoffe des Asthmas geeignet. Sie werden in Form von Tabletten oder Kapseln oder Spray in einer Dosis von 0,01-10 mg/kg Körpergewicht/Tag eingesetzt. Vorteilhaft können sie mit anderen bekannten Anti-asthmatika, z. B. Isoproterenol, Ephedrin usw., kombiniert werden.

Diese Verbindungen können weiterhin zur Behandlung von verschiedenen Hautkrankheiten, wie Psoriasis, aspezifischer und spezifischer Dermatitis, allergischen Ausschlägen, verwendet werden. Die Medikamentform ist in diesem Fall eine 0,5-4 % Wirkstoff enthaltende Salbe, Lösung oder ein Spray.

Die US-PS 42 58 199 betrifft ein Verfahren aus $PGF_{2\alpha}$- bzw. 9-Thio-$PGF_{2\alpha}$-Derivaten mit Phenylselenylhalogeniden und nachfolgender Oxydation Isoprostacyclin bzw. 6,9-Sulfoxa-prostacyclin und/oder 6,9-Sulfo-prostacyclin herzustellen. Diese Verbindungen weisen zwar eine höhere Stabilität auf, wirken auf den Blutdruck erhöhend und auf die Blutplättchenaggregation teilweise hemmend und teilweise sogar verstärkend.

Die Erfindung betrifft auch ein Verfahren zur Herstellung der optisch aktiven oder racemischen 5-substituierten $PGI_1$-Derivate der allgemeinen Formel I aus bicyclischen Hemiacetalderivaten der allgemeinen Formel II

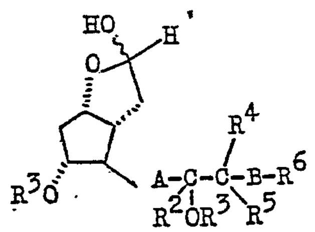

(II)

worin die Substituenten $R^2$, $R^3$, $R^4$, $R^5$, $R^6$, A und B die oben angegebene Bedeutung haben, durch Umsetzen mit eine aktive Methylengruppe enthaltenden Verbindungen der allgemeinen Formel III

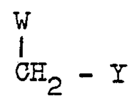

(III)

worin W und Y die oben angegebene Bedeutung haben.

Der Herstellung der Verbindungen der allgemeinen Formel I, liegt die in der Chemie der Prostacycline bisher noch nicht angewendete Knoevenagel-Kondensation zugrunde, die ausgehend von Verbindungen der allgemeinen Formel II in einem Schritt die $\alpha$-Kette der Prostacyclinanaloga und das bicyclische Produkt bildet.

In der Knoevenagel-Reaktion führt die Umsetzung von Oxo-Verbindungen mit Verbindungen, die eine aktive Methylengruppe enthalten, gewöhnlich $\beta$-Dicarbonylverbindungen, zu $\alpha,\beta$-ungesättigten Dicarbonylverbindungen (Berichte, 1896, *29,* 172 und J. Am. Chem. Soc. 1937, *59,* 2327).

Im erfindungsgemäßen Verfahren werden Verbindungen der allgemeinen Formel II als Moleküle, die eine Gruppe mit latenter Aldehydfunktion enthalten, mit Verbindungen der allgemeinen Formel III in Gegenwart des (der) in der Knoevenagel-Kondensation angewendeten Katalysators (Katalysatoren) umgesetzt. Die bei der Reaktion entstehenden $\alpha,\beta$-ungesättigten Dicarbonyl-, Bifunktions- und Nitroverbindungen können nicht isoliert werden, da die Reaktion mit der C-9-Hydroxyl-Gruppe des Prostaglandingerüsts sofort zu cyclischen Produkten der allgemeinen Formel I mit der gewünschten Struktur führt. Die Verbindungen der allgemeinen Formel I werden entweder direkt aus dem Reaktionsgemisch oder nach dessen basischer bzw. saurer Hydrolyse gewonnen.

Die bei der Synthese der Prostaglandine bisher noch nicht angewendete neue, konvergente, aus

einem Schritt bestehende Synthese ist nicht nur neu, sie bildet auch eine einfache, außerordentlich wirtschaftliche Synthese für die Herstellung der Verbindungen der allgemeinen Formel I. Bisher wurden Prostacyclinderivate mit ähnlicher Struktur aus $PGF_{2\alpha}$-Derivaten, die durch die Wittig-Reaktion aus Verbindungen der allgemeinen Formel II hergestellt wurden, durch Halocyclisierungsreaktion oder durch eine weitere, der elektrophilen Addition folgenden Reaktion hergestellt (J. Am. Chem. Soc., 1977, *99,* 2006).

Die Verbindungen der allgemeinen Formel II sind aus der Literatur bekannt (J. Am. Chem. Soc., 1969, *91,* 5675) bzw. sie können nach dem dort beschriebenen Verfahren aus den entsprechenden Ausgangsstoffen hergestellt werden. Ein Teil der Verbindungen der allgemeinen Formel III, wie β-Dicarbonylverbindungen oder eine doppelt aktivierte Methylengruppe enthaltende Verbindungen, sind auch bekannte Verbindungen (Patai S.: The Chemistry of the Carbonyl Group, Interscience Publ., London, 1966), die Herstellung der neuen Verbindungen der allgemeinen Formel III ist weiter unten beschrieben.

In der Knoevenagel-Reaktion werden meistens doppelt aktivierte Methylenkomponenten eingesetzt. Die elektronenanziehende Eigenschaft und die Fähigkeit der Nitrogruppe, Carbanion zu stabilisieren, sind an sich ausreichend zur Ausführung der Knoevenagel-Kondensation. Deshalb kann bei der Anwendung von Verbindungen der allgemeinen Formel III, in denen W eine Nitrogruppe bedeutet, auch mit Verbindungen der allgemeinen Formel III, in denen Z zwei Wasserstoffatome bedeuten, die erfindungsgemäße Reaktion ausgelöst werden.

Gemäß Erfindung werden die Verbindungen der allgemeinen Formel II — worin $R^3$ Wasserstoff oder eine der oben angegebenen Hydroxyl-Schutzgruppen bedeutet — mit Verbindungen der allgemeinen Formel III in An- oder Abwesenheit von Lösungsmitteln (wobei der Ueberschuß der Verbindungen der allgemeinen Formel III Lösungsmittel ist) in Gegenwart der in der Knoevenagel-Kondensation angewendeten Katalysatoren bei einer Temperatur zwischen 25-200 °C umgesetzt. Als Lösungsmittel werden vorteilhaft aromatische Kohlenwasserstoffe, Benzol, Toluol, Xylol eingesetzt, und die Reaktion wird bei einer dem Siedepunkt des Lösungsmittels entsprechenden Temperatur durchgeführt. Dann entweicht das bei der Reaktion entstehende Wasser, das mit dem Lösungsmittel ein azeotropes Gemisch bildet, und verschiebt das Gleichgewicht der Reaktionen in die gewünschte Richtung. Als Lösungsmittel können weitere, hinsichtlich der Reaktion inerte Lösungsmittel, wie Dimethylformamid, Dimethyl-sulfoxyd, Hexamethyl-phosphorsäure-triamid, auch chlorierte aliphatische und aromatische Kohlenwasserstoffe eingesetzt werden. Als Katalysator der Reaktionen können verschiedene Basen und Säuren bzw. deren Salze, vorteilhaft sekundäre Amine, Piperidin, Morpholin, Dialkyl-amine und mit organischen Säuren, vorteilhaft deren mit Essigsäure gebildeten Salze, verschiedene organische Säuren, vorteilhaft Essigsäure und Sulfonsäuren, Lewis-Säuren, vorteilhaft Bortrifluorid-äther, Zink[II]-chlorid, Titan[IV]-chlorid usw., weiterhin Aminosäuren verwendet werden.

Zweckmäßig wird so vorgegangen, daß die benzol- oder toluolhaltige Lösung der Verbindungen der allgemeinen Formel II mit 1-5 Äquivalenten der Verbindungen der allgemeinen Formel III in Gegenwart katalytischer oder von Fall zu Fall einer äquivalenten Menge Piperidinium-acetat oder Piperidin erhitzt wird, wobei je nach der Struktur der Verbindungen der allgemeinen Formel III nach einer Reaktionszeit von 2-72 Stunden mit fast quantitativer Konversion Verbindungen der allgemeinen Formel I erhalten werden, die weniger polar sind als die Verbindungen der allgemeinen Formel II.

Die Verbindungen der allgemeinen Formel I können durch säulenchromatographische Reinigung oder Kristallisation isoliert werden. Gewünschtenfalls können die durch basische oder saure Hydrolyse oder Reduktion erhaltenen Verbindungen der allgemeinen Formel I innerhalb des Begriffsbereichs der allgemeinen Formel I umgesetzt werden. Gewünschtenfalls werden durch Umsetzung von Verbindungen der allgemeinen Formel I, in denen $R^3$ Wasserstoff ist, mit einem Reagens, das die Hydroxylgruppe schützt, Verbindungen der allgemeinen Formel I erhalten, in der $R^3$ eine Hydroxyl-Schutzgruppe ist.

Ein weiterer Vorteil des erfindungsgemäßen Verfahrens besteht darin, daß Verbindungen der allgemeinen Formel I, in denen $R^3$ Wasserstoff ist, direkt aus Verbindungen der allgemeinen Formel II, in denen $R^3$ Wasserstoff bedeutet, und daß Verbindungen der allgemeinen Formel I, in denen $R^3$ eine Hydroxyl-Schutzgruppe ist, direkt aus Verbindungen der allgemeinen Formel II, in denen $R^3$ eine Hydroxyl-Schutzgruppe ist, gewonnen werden können.

In der Beschreibung sind unter $C_{1-4}$-Alkyl Methyl-, Äthyl-, n- und i-Propyl-, n-, i-, sec- und tert.-Butyl und unter $C_{1-4}$-Alkanoyl die, die aus den den oben angegebenen $C_{1-4}$-Alkylen entsprechenden Alkanen abgeleitet werden können, das sind Formyl, Acetyl, Propionyl und Butyryl, zu verstehen. Die $C_{1-6}$-Alkyle umfassen über die $C_{1-4}$-Alkyle hinaus auch unterschiedliche Pentyl- und Hexylgruppen.

Das Phenyl kann an beliebiger Stelle ein- oder mehrmals durch Halogen, durch eine Phenylgruppe, eine $C_{1-4}$-Alkylgruppe oder eine durch Halogen substituierte $C_{1-4}$-Alkylgruppe substituiert sein. Von den Phenyl-$C_{1-4}$-alkylgruppen wird der Benzylgruppe der Vorzug gegeben.

In der Beschreibung sind unter $C_{1-4}$-Alkyl-sulfenyl-Gruppe Gruppen zu verstehen, in denen die $C_{1-4}$-Alkylgruppe mit einem Schwefelatom verbunden ist. In den Phenyl-sulfenyl-Gruppen ist die Phenylgruppe mit einem Schwefelatom verbunden. Sie werden auch als $C_{1-4}$-Alkylthiogruppen bzw. als Phenylthiogruppen bezeichnet. In den $C_{1-4}$-Alkyl-sulfonyl-Gruppen bzw. den Phenyl-sulfonyl-Gruppen ist die Alkyl- bzw. Phenylgruppe mit einer —$SO_2$— Gruppe verbunden.

Charakteristische Vertreter für $C_{3-10}$-Cycloalkylgruppen sind die Cyclopropyl-, Cyclopentyl-, Cyclohexyl-, Cycloheptyl-, Cyclooctyl-, Cyclononyl- und Cyclodecyl-Gruppe.

Wenn in der Beschreibung nicht anders angegeben, ist unter einem pharmakologisch verträglichem Kation ein Äquivalent von Ionen mit der positiven Ladung 1, 2 oder 3 zu verstehen, die im lebenden Organismus bei den für die Verbindungen der Erfindung angegebenen Dosen keine unerwünschte Nebenwirkungen verursachen. Beispiele für solche Kationen sind die Ionen der Alkalimetalle, wie das Natrium-, Kalium- und das Lithiumion, die Ionen der Erdalkalimetalle, wie das Calcium- und Magnesiumion, das Aluminiumion, das Ammoniumion und die aus verschiedenen organischen Aminen ableitbaren ein- oder mehrwertigen Ammoniumionen, wie z. B. das Tris-hydroxymethylammoniumion.

Die Alkylgruppen der Tri-$C_{1-4}$-alkyl-silyl-Gruppen können identisch oder unterschiedlich sein. Die Heteroaryl-Gruppe kann ein oder mehrere Heteroatome, z. B. ein Stickstoff-, Sauerstoff- oder Schwefelatom an einer beliebigen Stelle des Rings enthalten.

Als besonders vorteilhaft haben sich folgende Verbindungen der Erfindung erwiesen.

5-Äthoxy-carbonyl-4-oxo-$PGI_1$-äthylester,
3α,β-(1'-Äthoxy-carbonyl-1'-cyano-methyl)-6β-(3S-hydroxy-oct-1E-enyl)-7α-Hydroxy-2-oxabicyclo-[3,3,0] octan,
3α,β-(1'-Äthoxy-carbonyl-2'-oxo-propyl)-6β-(3S-hydroxy-oct-1E-enyl)-7α-hydroxy-2-oxabicyclo-[3,3,0]octan,
3α,β-[1',1'-bis(Athoxycarbonyl)-methyl]-6β-(3S-hydroxy-oct-1E-enyl)-7α-hydroxy-2-oxabicyclo-[3,3,0]octan,
3α,β-(1'-Acetyl-2'-oxo-propyl)-6β-(3S-hydroxy-oct-1E-enyl)-7α-hydroxy-2-oxabicyclo[3,3,0]-octan,
3α,β-Nitro-methyl-6β-(3S-hydroxy-oct-1E-enyl)-7α-hydroxy-2-oxabicyclo[3,3,0]octan,
3α,β-Nitro-methyl-6β-(3R-acetoxy-oct-1E-enyl)-7α-acetoxy-2-oxabicyclo[3,3,0]octan,
5-Nitro-$PGI_1$-methylester,
5-Phenyl-sulfenyl-4-oxo-$PGI_1$-methylester,
5-p-Tolyl-sulfonyl-4-oxo-$PGI_1$-methylester,
5-Phenyl-sulfenyl-16,16-dimethyl-4-oxo-$PGI_1$-methylester,
5-p-Tolyl-sulfonyl-4-oxo-$PGI_1$-methylester-11,15-diacetat,
5-Nitro-$PGI_1$-dinatriumsalz,
5-Nitro-13,14-didehydro-$PGI_1$-methylester,
15-Epi-5-nitro-$PGI_1$-benzylester,
16-Phenoxy-17,18,19,20-tetranor-5-nitro-$PGI_1$-methylester,
2-Decarboxy-2-hydroxymethyl-5-nitro-20-äthyl-$PGI_1$,
5-Nitro-$PGI_1$-natriumsalz,
5-Nitro-13,14-didehydro-$PGI_4$-natriumsalz,
5-Phenyl-sulfenyl-13,14-didehydro-4-oxo-$PGI_1$-methylester,
5-Phenyl-sulfenyl-16-phenoxy-17,18,19,20-tetranor-4-oxo-$PGI_1$-methylester,
5-Phenyl-sulfenyl-16-(m-trifluormethyl-phenoxy)-17,18,19,20-tetranor-4-oxo-$PGI_1$-methylester,
5-Phenyl-sulfenyl-16-(m-chlor-phenoxy)-17,18,19,20-tetranor-4-oxo-$PGI_1$-methylester,
5-Phenyl-sulfenyl-13,14-didehydro-20-äthyl-4-oxo-$PGI_1$-methylester,
2,5-bis(Phenyl-sulfenyl)-4-oxo-$PGI_1$-methylester,
2,5-bis(Phenyl-sulfenyl)-13,14-didehydro-4-oxo-$PGI_1$-methylester,
5-Nitro-16-(m-chlor-phenoxy)-17,18,19,20-tetranor-$PGI_1$-methylester,
5-Nitro-16-(m-trifluormethyl-phenoxy)-17,18,19,20-tetranor-$PGI_1$-methylester.
5-Nitro-2-phenyl-sulfenyl-$PGI_1$-methylester,
5-Nitro-13,14-didehydro-2-phenyl-sulfenyl-$PGI_1$-methylester,
5-Nitro-13,14-didehydro-2-phenyl-sulfenyl-20-äthyl-$PGI_1$-methylester,
5-Nitro-2-phenyl-sulfenyl-16-phenoxy-17,18,19,20-tetranor-$PGI_1$-methylester,
5-Nitro-2-phenyl-sulfenyl-16-(m-chlor-phenoxy)-17,18,19,20-tetranor-$PGI_1$-methylester,
5-Nitro-2-phenyl-sulfenyl-16-(m-trifluormethyl-phenoxy)-17,18,19,20-tetranor-$PGI_1$-methylester.
5-Nitro-$\Delta^2$-$PGI_1$-methylester,
5-Nitro-13,14-didehydro-$\Delta^2$-$PGI_1$-methylester,
5-Nitro-13,14-didehydro-20-äthyl-$\Delta^2$-$PGI_1$-methylester,
5-Nitro-15-epi-$\Delta^2$-$PGI_1$-methylester,
5-Nitro-16-(m-chlor-phenoxy)-$\Delta^2$-17,18,19,20-tetranor-$PGI_1$-methylester
und
5-Nitro-16-(m-trifluormethyl-phenoxy)-$\Delta^2$-17,18,19,20-tetranor-$PGI_1$-methylester.

Weitere Einzelheiten der Erfindung werden von den Beispielen veranschaulicht, ohne die Erfindung darauf zu beschränken.

## Beispiel 1

5-Äthoxy-carbonyl-4-oxo-$PGI_1$-äthylester

(Formel I : W = Äthoxy-carbonyl, A = trans-Vinyl, Y = Z=C—X—Q, Z = Sauerstoff, X = $—CH_2—CH_2—$, Q = Äthoxy-carbonyl, $R^2$ = Wasserstoff mit β-Konfiguration, $R^3$ = Wasserstoff, $R^4$, $5^5$ = Wasserstoff, B = Methylen, $R^6$ = Propyl).

5,8 g (21,5 mMol) 3α,β-Hydroxy-6β-(3S-hydroxy-oct-1E-enyl)-7α-hydroxy-2-oxabicyclo[3,3,0]octan und 9,3 g (43 mMol) 3-Oxo-diäthyl-adipat (Formel III : W, Y, X, Z, Q wie oben angegeben) werden in 60 ml wasserfreiem Benzol gelöst und 4,3 ml Katalysatorlösung (die 42,8 g (0,5 Mol) Piperidin und 60 g (1 Mol) Essigsäure enthält und mit Benzol bis zu 1 000 ml ergänzt wurde) zugesetzt. Das Reaktionsgemisch wird auf einem vorgewärmten Ölbad nach dem Einfügen eines Soxhlet-Extrakteurs oder eines Wasserabscheideaufsatzes 1,5-2 Stunden erhitzt. Nachdem der Verlauf der Reaktion durch Dünnschichtchromatographie kontrolliert wurde (fast quantitative Konversion), wird das Gemisch mit 100 ml Äthylacetat verdünnt und der organische Teil wird 2x mit 20 ml Wasser, dann mit 20 ml gesättigter wäßriger Natriumchloridlösung gewaschen, über wasserfreiem Natriumsulfat getrocknet, filtriert und vom Lösungsmittel durch Destillation befreit. Die restlichen 14 g Rohprodukt werden auf 400 g Silicagel mit Äthylacetat der Chromatographie unterworfen. Es werden 9,2 g (91,5 %) der im Titel genannten Verbindung in Form eines farblosen Öls gewonnen.

$R_f$ : 0,72 mit dem Gemisch Äthylacetat-Methanol (Verhältnis 10 : 1) eluiert ;

IR (Film) : 3 350 (OH), 1 740, 1 725 (C = O), 965 $cm^{-1}$ (—CH=CH—) ;

$^1H$ NMR ($CDCl_3$) : δ5,5-5,6 (m, 2H, —CH=CH—, J = 15 Hz), 3,9-4,6 (4H CH—O, 4H $COOCH_2$—$CH_3$), 0,9 ppm (t, 3H, $CH_3$).

## Beispiel 2

3α,β-(1'-Äthoxy-carbonyl-1'-cyano-methyl)-6β-(3S-hydroxy-oct-1E-enyl)-7α-hydroxy-2-oxabicyclo-[3,3,0]octan

(Formel I : W = CN, Y = Äthoxy-carbonyl, A, B, $R^2$, $R^3$, $R^4$, $R^5$ und $R^6$ = wie in Verbindung von Beispiel 1).

2,7 g (20 mMol) 3α,β-Hydroxy-6β-(3S-hydroxy-oct-1E-enyl)-7α-hydroxy-2-oxabicyclo[3,3,0]octan und 2,26 g (20 mMol) Cyanessigsäure-äthylester werden in 20 ml wasserfreiem Benzol gelöst, und dann wird nach dem Zusatz von 1 ml Piperidiniumacetat-katalysatorlösung gemäß Beispiel 1 das Reaktionsgemisch mit dem Einfügen eines Wasserabscheideaufsatzes 2 Stunden erhitzt. Die Verarbeitung des Reaktionsgemisches erfolgt wie in Beispiel 1', und die als Eindampfrückstand gewonnenen 5 g Rohprodukt werden an 150 g Silicagel mit einem Gemisch von Äthylacetat-Hexan (Verhältnis 2 : 1) der Chromatographie unterworfen.

Es werden 3,4 g (92,5 %) der im Titel genannten Verbindung in Form eines farblosen Öls gewonnen.

$R_f$ : 0,5 mit Äthylacetat eluiert ;

IR (Film) : 3 350 (OH), 2 290 (C = N), 1 750 $cm^{-1}$ (C = O) ;

$^1H$ NMR ($CDCl_3$) : δ5,5-5,6 (m, 2H, —CH=CH—),
4-4,6 ppm (4H, CH—O, 2H $COOCH_2$—$CH_3$),
3,6 ppm (1H, NC—C<u>H</u>—COOEt).

## Beispiel 3

3α,β-(1'-Äthoxy-carbonyl-2'-oxo-propyl)-6β-(3S-hydroxy-oct-1E-enyl)-7α-hydroxy-2-oxabicyclo[3,3,0]octan

(Formel I : W = Äthoxy-carbonyl, Y = Acetyl, A, B, $R^2$, $R^3$, $R^4$, $R^5$ und $R^6$ = wie in Verbindung von Beispiel 1).

2,7 g (10 mMol) 3α,β-Hydroxy-6β-(3S-hydroxy-oct-1E-enyl)-7α-hydroxy-2-oxabicyclo[3,3,0]octan und 2,6 g (20 mMol) Acetessigsäure-äthylester werden in 20 ml wasserfreiem Benzol gelöst und 0,8 ml der Piperidiniumacetat-katalysatorlösung nach Beispiel 1 zugesetzt. Dann wird das Reaktionsgemisch nach dem Einfügen eines Wasserabscheideaufsatzes 3 Stunden erhitzt. Die weitere Aufarbeitung des Reaktionsgemisches erfolgt wie in Beispiel 1, das als Eindampfrückstand erhaltene Rohprodukt wird an 150 g Silicagel mit Äthylacetat der Chromatographie unterworfen, wobei 3,25 g (85 %) der im Titel genannten Verbindung in Form eines farblosen Öls erhalten werden.

$R_f$ : 0,55 mit einem Gemisch von Äthylacetat-Methanol (Verhältnis 10 : 1) eluiert ;

IR (Film) : 3 350 (OH), 1 740, 1 720 $cm^{-1}$ (C = O) ;

$^1H$ NMR ($CDCl_3$) : δ5,5-5,6 (m, 2H, —CH=CH—),
4-4,6 (4H, CH—O, 2H $COOCH_2$—$CH_3$),
2,1 ppm (s, 3H, O=C—$CH_3$).

## Beispiel 4

3α,β[1',1'-bis(Äthoxy-carbonyl)-methyl]-6β-(3S-hydroxy-oct-1E-enyl)-7α-hydroxy-2-oxabicyclo[3,3,0]octan

(Formel I : W und Y = Äthoxy-carbonyl, A, B, $R^2$, $R^3$, $R^4$, $R^5$ und $R^6$ = wie in Verbindung von Beispiel 1).

2,7 g (10 mMol) 3α,β-Hydroxy-6β-(3S-hydroxy-oct-1E-enyl)-7α-hydroxy-2-oxabicyclo[3,3,0]octan werden unter leichtem Erwärmten in 8 g (50 mMol) Malonsäure-diäthylester gelöst und der farblosen Lösung nach Abkühlen auf Raumtemperatur 11 ml (11 mMol) Piperidin zugesetzt. Dann wird das Reaktionsgemisch 24 Stunden bei Raumtemperatur stehen gelassen. Nach Beendigung der Reaktion (Kontrolle durch Dünnschichtchromatographie) wird das Reaktionsgemisch ohne Verarbeitung auf 300 g Silicagel mit Äthylacetat der Chromatographie unterworfen, die Fraktionen, die das Reaktionsprodukt enthalten, werden vereinigt und vom Lösungsmittel durch Destillation befreit.

Es werden 3,3 g (80 %) der im Titel genannten Verbindung in Form eines farblosen Öls gewonnen.

$R_f$ : 0,57 mit Äthylacetat eluiert ;
IR (Film) : 3 300 (OH), 1 740-1 750 $cm^{-1}$ (C = O) ;
$^1H$ NMR ($CDCl_3$) : δ5,5-5,6 (m, 2H, —CH=CH—),
4-4,6 ppm (4H, CH—O, 4H $COOCH_2$).

Beispiel 5

3α,β-(1'-Acetyl-2'-oxo-propyl)-6β-(3S-hydroxy-oct-1E-enyl)-7α-hydroxy-2-oxabicyclo[3,3,0]octan
(Formel I : W und Y = Acetyl, A, B, $R^2$, $R^3$, $R^4$, $R^5$ und $R^6$ = wie in Verbindung von Beispiel 1).

270 mg (1 mMol) 3α,β-Hydroxy-6β-(3S-hydroxy-oct-1E-enyl)-7α-hydroxy-2-oxabicyclo[3,3,0]octan werden unter leichtem Erwärmen in 2 g (20 mMol) Acetylaceton gelöst und nach Abkühlen auf Raumtemperatur 0,11 ml (1,1 mMol) Piperidin zugesetzt. Dann wird das Reaktionsgemisch 10 Stunden bei Raumtemperatur gerührt. Nach Beendigung der Reaktion wird das Reaktionsgemisch ohne Verarbeitung auf 40 g Silicagel mit Äthylacetat der Chromatographie unterworfen, wobei die Fraktionen, die das Reaktionsprodukt enthalten, vereinigt und vom Lösungsmittel durch Destillation befreit werden.

Es werden 282 mg (80 %) der im Titel genannten Verbindung in Form eines farblosen Öls gewonnen.

$R_f$ : 0,7 mit dem Gemisch von Äthylacetat-Methanol (Verhältnis 10 : 1) eluiert ;
IR (Film) : 3 350, 1 720, 1 700 $cm^{-1}$ (C = O) ;
$^1H$ NMR ($CDCl_3$) : δ5,5-5,6 (m, 2H, —CH=CH—),
4-4,6 ppm (4H, CH—O),
2,2-2,3 ppm (6H, ss, O=C—$CH_3$).

Beispiel 6

3α,β-Nitro-methyl-6β-(3S-hydroxy-oct-1E-enyl)-7α-hydroxy-2-oxabicyclo[3,3,0]octan

2,0 g (7,4 mMol) 3α,β-Hydroxy-6β-(3S-hydroxy-oct-1E-enyl)-7α-hydroxy-2-oxabicyclo[3,3,0]octan werden in 20 ml Nitro-methan gelöst und 0,5 ml (5,1 mMol) Piperidin zugesetzt. Dann wird das Reaktionsgemisch 12 Stunden bei Raumtemperatur gerührt. Nach dem Verdünnen mit 100 ml Äthylacetat wird die organische Lösung 2x mit 30 ml Natriumhydrogen-sulfat-lösung, mit 20 ml Wasser, dann mit 20 ml gesättigter wäßriger Natriumchlorid-lösung gewaschen, über Natriumsulfat getrocknet, filtriert und vom Lösungsmittel durch Destillation befreit. Das restliche Rohprodukt wird auf 80 g Silicagel mit Äthylacetat der Chromatographie unterworfen.

Es werden 2,0 g (86,3 %) der im Titel genannten Verbindung in Form eines schwachgelben Öls gewonnen.

$R_f$ : 0,48 mit dem Gemisch von Äthylacetat-Aceton (Verhältnis 10 : 1) eluiert ;
IR (Film) : 1 560 $cm^{-1}$ ($NO_2$) ;
$^1H$ NMR ($CDCl_3$) : δ5,4-5,5 (m, 2H, —CH=CH—),
4,5 ppm (m, 2H, $CH_2$—$NO_2$).

Beispiel 7

3α,β-Nitro-methyl-6β-(3R-acetoxy-oct-1E-enyl)-7α-acetoxy-2-oxabicyclo[3,3,0]octan
(Formel I : W = Nitro, Y = Wasserstoff, $R^2$ = Wasserstoffatom mit α-Konfiguration, $R^3$ = Acetyl, A, B, $R^4$, $R^5$ und $R^6$ = wie in Verbindung von Beispiel 1).

168 mg (0,47 mMol) 3α,β-Hydroxy-6β-(3R-acetoxy-oct-1E-enyl)-7α-acetoxy-2-oxabicyclo[3,3,0]octan löst man in 1,7 ml Nitromethan und rührt nach dem Zusatz von 50 μl (0,5 mMol) Piperidin das Reaktionsgemisch 10 Stunden bei Raumtemperatur. Nach dem Verdünnen mit 20 ml Äthylacetat wird der organische Teil mit 5 ml Natriumhydrogen-sulfat-lösung, 5 ml Wasser, dann mit 5 ml gesättigter wäßriger Natriumchlorid-lösung gewaschen, über Natriumsulfat getrocknet und dann vom Lösungsmittel durch Destillation befreit. Das als Rückstand erhaltene Rohprodukt wird auf 15 g Silicagel mit dem Gemisch von Hexan-Äthylacetat (Verhältnis 2 : 1) der Chromatographie unterworfen.

Es werden 160 mg (90 %) der im Titel genannten Verbindung in Form eines farblosen Öls erhalten.

$R_f$ : 0,27 mit dem Gemisch von Hexan-Äthylacetat (Verhältnis 2 : 1) eluiert ;
IR (Film) : 1 560 ($NO_2$), 1 740 $cm^{-1}$ (C = O).

Beispiel 8

5-Nitro-$PGI_1$-methylester

(Formel I: W = Nitro, Y = Z=C—X—Q, Z = zwei Wasserstoffe, X = $—CH_2—CH_2—$, Q = Methoxycarbonyl, die übrigen Substituenten entsprechen der im Titel von Beispiel 1 genannten Verbindung).

270 mg (1 mMol) 3α,β-Hydroxy-6β-(3S-hydroxy-oct-1E-enyl)-7α-hydroxy-2-oxabicyclo[3,3,0]octan werden in 2,7 ml (17 mMol) 5-Nitro-valeriansäure-methylester der allgemeinen Formel III (W = Nitro, Y = Z=C—X—Q, Z = zwei Wasserstoffe, X = $—CH_2—CH_2$, Q = methoxy-carbonyl) gelöst und 0,2 ml (2 mMol) Piperidin zugesetzt. Dann wird das Reaktionsgemisch 72 Stunden bei 60 °C gerührt, danach mit 40 ml Äthylacetat verdünnt, 2x mit 10 ml gesättigter Natriumhydrogen-sulfat-lösung, 10 ml Wasser, 10 ml gesättigter wäßriger Salzlösung gewaschen, über Natriumsulfat getrocknet und vom Lösungsmittel durch Destillation befreit. Das als Eindampfrückstand gewonnene Rohprodukt wird auf 40 g Silicagel mit Äthylacetat der Chromatographie unterworfen, wobei 358 mg (86,2 %) der im Titel genannten Verbindung in Form eines farblosen Öls erhalten werden.

$R_f$ : 0,41 mit dem Gemisch von Äthylacetat-Aceton (Verhältnis 10 : 1) eluiert ;
IR (Film) : 1 560 ($NO_2$), 1 740 $cm^{-1}$ (C = O) ;
$^1H$ NMR ($CDCl_3$) : δ5,5-5,6 (m, 2H, —CH=CH—),
3,67 (s, 3H, $COOCH_3$),
3,6-4,6 (4H, CH—O, 1H $CH—NO_2$).

Beispiel 9

5-Phenyl-sulfenyl-4-oxo-$PGI_1$-methylester

(Formel I: W = $C_6H_5—S$, Q = Methoxycarbonyl, die übrigen Substituenten, wie in Verbindung von Beispiel 1).

Das 920 mg (3,4 mMol) 3α,β-Hydroxy-6β-(3S-hydroxy-oct-1E-enyl)-7α-hydroxy-2-oxabicyclo[3,3,0]octan, 1, 64 g (6,88 mMol) 5-Phenyl-sulfenyl-4-oxo-valeriansäure-methylester, 5 ml Benzol und 7 ml 0,5 molare benzolhaltige Piperidiniumacetat-lösung enthaltende Reaktionsgemisch wird unter Anwendung eines Wasserabscheideaufsatzes 60 Stunden erhitzt. Nach Beendigung der Reaktion (Kontrolle durch Dünnschichtchromatographie) werden dem Gemisch 100 ml Äthylacetat zugesetzt. Dann wird das Gemisch 2x mit 10 ml Wasser, 10 ml gesättigter wäßriger Natriumchloridlösung gewaschen, über Magnesiumsulfat getrocknet und dann vom Lösungsmittel durch Destillation befreit. Das Rohprodukt wird auf 30 g Silicagel bei einem Überdruck von 1,5 bar mit einem Äthylacetat-Eluent der Chromatographie unterworfen, wobei 1,4 g (84 %) der im Titel genannten Verbindung in Form eines farblosen Öls gewonnen werden.

$R_f$ : 0,46 mit einem Gemisch von Äthylacetat-Aceton (Verhältnis von 1 : 1) eluiert ;
IR (Film) : 1 750, 1 710 $cm^{-1}$ (C = O) ;
$^1H$ NMR ($CDCl_3$) : δ7,4-7,6 (3H, m, aromatisch),
7,0-7,2 (2H, m, aromatisch),
5,6-5,7 (2H, m, —CH=CH—),
3,4-4,6 (7H, 2H austauschbar durch $D_2O$),
3,45 ppm (3H, s, $COOCH_3$).

Beispiel 10

5-p-Tolyl-sulfonyl-4-oxo-$PGI_1$-methylester

(Formel I: W = p-Tolyl-sulfonyl, Q = Methoxycarbonyl, die übrigen Substituenten, wie in Verbindung von Beispiel 1).

20 ml benzolhaltiger Suspension von 500 mg (1,85 mMol) 3α,β-Hydroxy-6β-(3S-Hydroxy-oct-1E-enyl)-7α-hydroxy-2-oxabicyclo[3,3,0]octan werden 1,31 g (4,88 mMol) 5-p-Tolyl-sulfonyl-4-oxo-valeriansäure-methylester zugesetzt. Dann wird das Reaktionsgemisch nach der Zugabe von 1,5 ml 0,5 molarer benzolhaltiger Piperidiniumacetatlösung unter Anwendung eines Wasserabscheideaufsatzes 36 Stunden erhitzt, danach mit 50 ml Äthylacetat verdünnt, nacheinander 2x mit 10 ml Wasser, 10 ml gesättigter wäßriger Natriumchloridlösung gewaschen, über Magnesiumsulfat getrocknet und von dem Lösungsmittel durch Destillation befreit. Das Rohprodukt wird auf 25 g Silicagel bei einem Überdruck von 1,5 bar mit Äthylacetat der Chromatographie unterworfen, wobei 886 mg (89,3 %) der im Titel genannten Verbindung in Form eines farblosen Öls erhalten werden.

$R_f$ : 0,48 mit dem Gemisch von Äthylacetat-Aceton (Verhältnis 1 : 1) eluiert ;
IR (Film) : 1 720, 1 750 $cm^{-1}$ (C = O) ;
$^1H$ NMR ($CDCl_3$) : δ7,1-7,4 (4H, aromatisch),
3,5-4,5 (5H, m),
3,67 (3H, s, $COOCH_3$),
2,47 ppm (3H, s).

## Beispiel 11

5-Phenyl-sulfenyl-16,16-dimethyl-4-oxo-$PGI_1$-methylester
(Formel I : W = $C_6H_5$-S-, $R^4$ und $R^5$ = Methyl, Q = Methoxycarbonyl die übrigen Substituenten, wie in Verbindung von Beispiel 1).

20 ml toluolhaltiger Lösung von 1,2 g (4 mMol) 3α, β-Hydroxy-6β-(3S-hydroxy-4,4-dimethyl-oct-1E-enyl)-7α-hydroxy-2-oxabicyclo [3,3,0] octan werden 2,86 g (12 mMol) 5-Phenyl-sulfenyl-4-oxo-valeriansäure-methylester und 10 ml 0,5 molare benzolhaltige Piperidiniumacetatlösung zugesetzt. Dann wird das Reaktionsgemisch 20 Stunden unter Anwendung eines Wasserabscheideaufsatzes erhitzt, danach mit 100 ml Äthylacetat verdünnt, 2x mit 10 ml Wasser, 10 ml gesättigter wäßriger Natriumchloridlösung gewaschen, über Magnesiumsulfat getrocknet und vom Lösungsmittel durch Destillation befreit. Das Rohprodukt wird auf Silicagel mit Äthylacetat der Chromatographie unterworfen.

Es werden 1,66 g (80 %) der im Titel genannten Verbindung in Form eines blaßgelben Öls gewonnen.

$R_f$ : 0,52 mit Äthylacetat eluiert ;
IR (Film) : 1 745, 1 710 $cm^{-1}$ (C=O) ;
$^1H$ NMR ($CDCl_3$) : δ7,4-7,6 (3H, m),
7,0-7,2 (2H, m),
5,5-5,6 (2H, m),
3,57 ppm (3H, s).

## Beispiel 12

5-p-Tolyl-sulfonyl-4-oxo-$PGI_1$-methylester-11,15-diacetat
(Formel I : W = p-Tolyl-sulfonyl-, $R^3$ = Acetyl, Q = Methoxycarbonyl-, die übrigen Substituenten entsprechen der im Titel von Beispiel 1 genannten Verbindung).

30 ml benzolhaltiger Lösung von 1 g (1,86 mMol) 5-p-Tolyl-sulfonyl-4-oxo-$PGI_1$-methylester (Verbindung des Beispiels 10) werden 3 ml Pyridin und 0,53 ml (5,6 mMol) Essigsäureanhydrid zugesetzt. Die Reaktion ist bei Raumtemperatur innerhalb von 12 Stunden beendet. Dann setzt man dem Reaktionsgemisch 20 ml Wasser zu und rührt 10 Minuten. Dann wird nach dem Zusatz von 150 ml Äther die organische Phase mit 5 ml 5n Salzsäure, 20 ml 1n Salzsäure, 20 ml Wasser, 20 ml gesättigter Natriumhydrogen-carbonatlösung, 2x mit 20 ml Wasser und schließlich mit 20 ml gesättigter wäßriger Natriumchloridlösung gewaschen, über Magnesiumsulfat getrocknet, filtriert, und vom Lösungsmittel durch Destillation befreit. Nach kurzer Säulenchromatographie mit einem Gemisch von Äthylacetat - Hexan (Verhältnis 1 : 1) als Eluent werden 1,02 g (88 %) der im Titel genannten Verbindung in Form eines farblosen Öls erhalten.

$R_f$ : 0,31 mit dem Gemisch von Hexan — Äthylacetat (Verhältnis 1 : 1) eluiert ;
$^1H$ NMR ($CDCl_3$) : δ5,52 (2H, m),
5,15 (1H, m),
4,0-4,9 (4H, m),
3,65 (3H, s),
1,9-2,05 ppm (3H ; s ; 3H, s).

## Beispiel 13

5-Phenyl-sulfenyl-4-oxo-valeriansäure-methylester

Zu der Lösung von 100 ml 1 molaren methanolhaltigem Natrium-thio-phenolat setzt man in 40 Minuten 22 g (0,1 Mol) 5-Brom-4-oxo-valeriansäure-methylester zu, rührt das Reaktionsgemisch 2 Stunden bei Raumtemperatur und destilliert das Methanol im Vakuum ab. Der Rückstand wird in 200 ml Äthylacetat aufgenommen, mit 30 ml Wasser, 30 ml Natriumhydrogen-carbonatlösung, 30 ml gesättigter Salzlösung gewaschen, über Magnesiumsulfat getrocknet, filtriert und vom Lösungsmittel durch Destillation befreit. Mit Säulenchromatographie (Hexan-Äthylacetat 4 : 1) werden 21,3 g (85 %) der im Titel genannten Verbindung in Form eines farblosen Öls gewonnen. Siedepunkt : 128-130 °C/0,2 Hgmm.

$^1H$ NMR ($CDCl_3$) : δ7,2 (5H, m),
3,68 (2H, s),
3,56 (3H, s),
2,3-3,1 ppm (4H, m).

## Beispiel 14

5-Nitro-$PGI_1$-Dinatriumsalz
(Formel I : Y = Z=C—X—Q, Z = zwei Wasserstoffe,
X = $—CH_2—CH_2—$, Q = —COONa, die übrigen Substituenten entsprechen der im Titel von Beispiel 1 genannten Verbindung).

116,15 mg (0,28 mMol) 5-Nitro-$PGI_1$-methylester (Verbindung des Beispiels 8) löst man in 1 ml Methanol, setzt 0,45 ml (1,12 mMol) 2,5n Natrium-methoxyd zu, rührt das Gemisch 2 Stunden bei Raumtemperatur, versetzt die Lösung mit 10 µl (0,56 mMol) Wasser und rührt sie 12 Stunden bei Raumtemperatur. In kleinen Anteilen setzt setzt man dem Reaktionsgemisch 10 ml Acetonitril zu, filtriert den ausgefällten gelben kristallinen Stoff ab und trocknet ihn bei Raumtemperatur, wobei man 120 mg der im Titel genannten Verbindung in Form eines gelbbraunen kristallinen Stoffes erhält.

Schmelzpunkt : Zerfall > 200 °C,

IR (KCl) : 3 450 $cm^{-1}$ OH ; 1 580, 1 430 $cm^{-1}$ $COO^-$ ; 965 $cm^{-1}$ —CH=CH— ;

$^1H$ NMR ($D_2O$) : δ5,6 ppm (2H, —C$\underline{H}$=C$\underline{H}$—,)
3,5-5 ppm (4H, —O—C$\underline{H}$—),
0,9 ppm 3H, (—$CH_3$),
1-3 ppm (20 $\underline{H}$—).

## Beispiel 15

13,14-Didehydro-5-nitro-$PGI_1$-methylester
(Formel I : A = Äthinylen, die übrigen Substituenten, wie in Beispiel 8).

268 mg (1 mMol) 3α,β-Hydroxy-6β-(3S-hydroxy-octinyl)-7α-hydroxy-2-oxabicyclo [3,3,0]octan (A = Äthinylen, die übrigen Substituenten, wie in Beispiel 1), werden in 5 ml Benzol gelöst und 85 mg (1 mMol) Piperidin, 10 mg (0,17 mMol) Essigsäure sowie 1,61 g (10 mMol) 5-Nitro-valeriansäure-methylester zugesetzt. Das Reaktionsgemisch wird 48 Stunden bei 60 °C gerührt. Die weitere Aufarbeitung erfolgt wie in Beispiel 8. Das erhaltene Rohprodukt wird auf 40 g Silicagel mit dem Gemisch von Äthylacetat - Hexan (Verhältnis 2 : 1) der Chromatographie unterworfen, wobei man 309 mg (75 %) der im Titel genannten Verbindung in Form eines farblosen Öls erhält.

$R_f$ : 0,54 mit Äthylacetat eluiert ;

IR (Film) : 1 735 (C=O), 1 560 $cm^1$ ($NO_2$) ;

$^1H$ NMR ($CDCl_3$) : δ4-4,6 (m, 5H, —CH—O, CH—$NO_2$),
3,68 (s, 3H, $COOCH_3$).

## Beispiel 16

15-Epi-5-nitro-$PGI_1$-benzylester
(Formel I : Q = Benzyloxy-carbonyl, $R^2$ = Wasserstoff mit α-Konfiguration, die übrigen Substituenten, wie in Beispiel 8).

2,7 g (10 mMol) 3α,β-Hydroxy-6β-(3R-hydroxy-oct-1E-enyl)-7α-hydroxy-2-oxabicyclo [3,3,0] octan ($R^2$ = Wasserstoff mit α-Konfiguration, die übrigen Substituenten, wie in Beispiel 1) werden in 40 ml Benzol gelöst und 850 mg (10 mMol) Piperidin, 120 mg (2 mMol) Essigsäure sowie 11,2 g (50 mMol) 5-Nitro-valeriansäure-benzylester zugesetzt. Die weitere Aufarbeitung erfolgt wie in Beispiel 8. Das Rohprodukt wird auf 300 g Silicagel mit dem Gemisch von Äthylacetat-Hexan (Verhältnis 4 : 1) der Chromatographie unterworfen, wobei 3,47 g (72 %) der im Titel genannten Verbindung in Form eines farblosen Öls erhalten werden.

$R_f$ : 0,28 mit Äthylacetat eluiert ;

IR (Film) : 1 745 (C=O), 1 560 $cm^{-1}$ ($NO_2$) ;

$^1H$ NMR ($CDCl_3$) : δ7,4 (s, 5H, aromatisch),
5,56 (m, 2H, —CH=CH),
5,15 (s, 2H, $CO_2$—$CH_2$),
3,7-4,6 (m, 5H).

## Beispiel 17

16-Phenoxy-17,18,19,20-tetranor-5-nitro-$PGI_1$-methylester
(Formel I : W = Nitro B = Sauerstoff, $R^6$ = Phenyl, die übrigen Substituenten, wie in Beispiel 9).

610 mg (2 mMol) 3α,β-Hydroxy-6β-(3S-hydroxy-4-phenoxy-but-1E-enyl)-7α-hydroxy-2-oxabicyclo [3,3,0] octan (B = Sauerstoff, $R^6$ = Phenyl, die übrigen Substituenten, wie in Beispiel 9) werden in 10 ml Benzol gelöst und 170 mg (2 mMol) Piperidin, 20 mg (0,34 mMol) Essigsäure sowie 3,2 g (20 mMol) 5-Nitro-valeriansäure-methylester zugesetzt. Das Reaktionsgemisch wird 48 Stunden bei 60 °C gerührt. Die weitere Aufarbeitung erfolgt wie in Beispiel 8. Das Rohprodukt wird auf 70 g Silicagel mit dem Gemisch von Äthylacetat-Hexan (Verhältnis 4 : 1) der Chromatographie unterworfen, wobei 718 mg (80 %) der im Titel genannten Verbindung in Form eines farblosen Öls erhalten werden.

$R_f$ : 0,47 mit dem Gemisch von Äthylacetat-Aceton (Verhältnis 10 : 1) eluiert ;

IR (Film) : 1 745 (C=O), 1 562 $cm^{-1}$ ($NO_2$) ;

$^1H$ NMR ($CDCl_3$) : δ7,4-7,1 (m, 5H, aromatisch),
5,5-5,6 (m, 2H, —CH=CH),
3,7-4,8 (m, 7H),
3,68 (s, 3H, $COOCH_3$).

Beispiel 18

2-Decarboxy-2-hydroxymethyl-20-äthyl-5-nitro-$PGI_1$
(Formel I : W = Nitro, Q = Hydroxy-methyl, $R^6$ = Pentyl, die übrigen Substituenten, wie in Beispiel 9).

300 mg (1 mMol) 3α,β-Hydroxy-6β-(3S-hydroxy-dec-1E-enyl)-7α-hydroxy-2-oxabicyclo [3,3,0] octan ($R^6$ = Pentyl, die übrigen Substituenten, wie in Beispiel 1) werden in 5 ml Benzol gelöst und 85 mg (1 mMol) Piperidin, 10 mg (0,17 mMol) Essigsäure sowie 1,33 g (10 mMol) 5-Nitro-pentanol zugesetzt. Das Reaktionsgemisch wird 48 Stunden bei 60 °C gerührt. Die weitere Aufarbeitung erfolgt wie in Beispiel 8. Das Rohprodukt wird mit dem Gemisch von Äthylacetat-Aceton (Verhältnis 10 : 1) der Chromatographie unterworfen, wobei 318 mg (82 %) der im Titel genannten Verbindung in Form eines farblosen Öls gewonnen werden.

$R_f$ : 0,45 mit dem Gemisch von Äthylacetat-Aceton (Verhältnis 3 : 1) eluiert ;
IR (Film) : 3 450 (OH), 1 560 $cm^{-1}$ ($NO_2$) ;
$^1H$ NMR ($CDCl_3$) : δ5,5-5,6 (m, 2H, —CH=CH),
3,7-4,8 (m, 7H),
0,91 (t, 3H, $CH_3$).

Beispiel 19

5-Nitro-$PGI_1$-Natriumsalz

978 mg (2,37 mMol) 5-Nitro-$PGI_1$-methylester löst man in dem Gemisch von 7,1 ml (7,1 mMol) 1n Natriumhydroxyd und 7 ml Methanol, rührt das Reaktionsgemisch 24 Stunden bei Raumtemperatur, dampft das Methanol im Vakuum ab und setzt der restlichen wäßrigen Lösung 20 ml Acetonitril zu. Das kristallin ausfallende Produkt wird abfiltriert, mit Äther gewaschen und über Calciumchlorid getrocknet. Es werden 1,05 g (100 %) der im Titel genannten Verbindung in Form von weißen Kristallen erhalten.

$R_f$ : 0,26 mit dem Gemisch von Benzol-Dioxan-Essigsäure (Verhältnis 20 : 10 : 1) eluiert ;
IR (KBr) : 3 450, 1 500, 1 440, 1 330, 1 170, 1 050, 890, 830 $cm^{-1}$ ;
$^1H$ NMR (DMSO-$d_6$) : δ5,5 (m, 2H, —CH=CH),
3,9-4,8 (m, 4H, —O—CH).

Beispiel 20

13,14-Didehydro-5-nitro-$PGI_1$-Natriumsalz

88 mg (0,214 mMol) 13,14-Didehydro-5-nitro-$PGI_1$-methylester löst man in 0,7 ml Methanol und versetzt die Lösung mit 0,64 ml (0,64 mMol) 1n Natriumhydroxydlösung. Die weitere Aufarbeitung erfolgt wie in Beispiel 19, wobei 95 mg (100 %) der im Titel genannten Verbindung in Form von weißen Kristallen erhalten werden.

$R_f$ : 0,38 mit dem Gemisch von Benzol-Dioxan-Essigsäure (Verhältnis 20 : 10 : 1) eluiert ;
IR (KBr) : 3 450, 2 250, 1 435, 1 320, 1 145, 900 $cm^{-1}$ ;
$^1H$ NMR (DMSO-$d_6$) : δ3,7-4,8 (m, 4H),
0,93 (t, 3H—$CH_3$).

In analoger Weise werden ausgehend von Verbindungen der allgemeinen Formeln II und III folgende Verbindungen der allgemeinen Formel I hergestellt :

5-Phenyl-sulfenyl-13,14-didehydro-4-oxo-$PGI_1$-methyl-ester
$R_f$ : 0,56 Äthylacetat-Aceton 1 : 1 (W = $C_6H_5$—S—),
5-Phenyl-sulfenyl-16-phenoxy-17,18,19,20-tetranor-4-oxo-$PGI_1$-methylester
$R_f$ : 0,53 Athylacetat-Aceton 1 : 1 (W = $C_6H_5$—S—),
5-Phenyl-sulfenyl-16-(m-trifluormethyl-phenoxy)-17,18,19,20-tetranor-4-oxo-$PGI_1$-methylester
$R_f$ : 0,4 Äthylacetat — Aceton 3 : 1 (W = $C_6H_5$—S—),
5-Phenyl-sulfenyl-16-(m-chlor-phenoxy)-17,18,19,20-tetranor-4-oxo-$PGI_1$-methylester
$R_f$ : 0,29 Äthylacetat (W = $C_6H_5$—S—),
5-Phenyl-sulfenyl-13,14-didehydro-20-äthyl-4-oxo-$PGI_1$-methylester
$R_f$ : 0,58 Äthylacetat-Aceton 1 : 1 (W = $C_6H_5$—S—),
2,5-bis-(Phenyl-sulfenyl)-4-oxo-$PGI_1$-methylester
$R_f$ : 0,4 Äthylacetat-Aceton 1 : 1 (W = $C_6H_5$—S— und X = —$CH_2$—CH—S—$C_6H_5$),
2,5-bis-(Phenyl-sulfenyl)-13,14-didehydro-4-oxo-$PGI_1$-methylester
$R_f$ : 0,49 Äthylacetat-Aceton 1 : 1 (W = $C_6H_5$—S— und X = —$CH_2$—CH—S—$C_6H_5$),
sowie die aus diesen Verbindungen durch Hydrolyse erhaltenen Salze und Säuren.
16-(m-Chlor-phenoxy)-17,18,19,20-tetranor-5-nitro-$PGI_1$-methylester
$R_f$ : 0,5 Äthylacetat,
16-(m-Trifluormethyl-phenoxy)-17,18,19,20-tetranor-5-nitro-$PGI_1$-methylester
$R_f$ : 0,54 Aceton-Hexan 1 : 1,

2-Phenyl-sulfenyl-5-nitro-$PGI_1$-methylester
$R_f$ : 0,48 Äthylacetat-Aceton 10 : 1 (X = —$CH_2$—CH—S—$C_6H_5$),
2-Phenyl-sulfenyl-13,14-didehydro-5-nitro-$PGI_1$-methylester
$R_f$ : 0,55 Äthylacetat-Aceton 10 : 1 (X = —$CH_2$—CH—S—$C_6H_5$),
2-Phenyl-sulfenyl-13,14-didehydro-20-äthyl-5-nitro-$PGI_1$-methylester
$R_f$ : 0,41 Äthylacetat, (X = —$CH_2$—CH—S—$C_6H_5$),
2-Phenyl-sulfenyl-16-phenoxy-17,18,19,20-tetranor-5-nitro-$PGI_1$-methylester
$R_f$ : 0,40 Äthylacetat, (X = —$CH_2$—CH—S—$C_6H_5$),
2-Phenyl-sulfenyl-16-(m-chlor-phenoxy)-17,18,19,20-tetranor-5-nitro-$PGI_1$-methylester
$R_f$ : 0,39 Äthylacetat-Aceton 4 : 1, (X = —$CH_2$—CH—S—$C_6H_5$),
2-Phenyl-sulfenyl-16-(m-trifluormethyl-phenoxy)-17,18,19,20-tetranor-5-nitro-$PGI_1$-methylester
$R_f$ : 0,38 Äthylacetat, (X = —$CH_2$—CH—S—$C_6$—$H_5$),
5-Nitro-$\Delta^2$—$PGI_1$-methylester
$R_f$ : 0,33 Äthylacetat, (X = —CH = CH—),
5-Nitro-13,14-didehydro-$\Delta^2$-$PGI_1$-methylester
$R_f$ : 0,39 Äthylacetat, (X = —CH = CH—),
5-Nitro-13,14-didehydro-20-äthyl-$\Delta^2$-$PGI_1$-methyl-ester
$R_f$ : 0,42 Äthylacetat, (X = —CH = CH—),
5-Nitro-15-epi-$\Delta^2$-$PGI_1$-methylester
$R_f$ : 0,40 Äthylacetat-Aceton 3 : 1, (X = —CH = CH—),
16-(m-Chlor-phenoxy)-17,18,19,20-tetranor-5-nitro-$\Delta^2$-$PGI_1$-methylester
Rf : 0,33 Äthylacetat-Aceton 4 : 1, (X = —CH = CH—),
16-(m-Trifluormethyl-phenoxy)-17,18,19,20-tetranor-5-nitro-$\Delta^2$-$PGI_1$-methylester
$R_f$ : 0,36 Äthylacetat, (X = —CH = CH—).

Die in der Beschreibung angeführten $R_f$-Werte wurden auf einer Merck Kieselgel 60 $F_{254}$-Platte bestimmt.

**Patentansprüche**

1. Optisch aktive und racemische 5-substituierte $PGI_1$-Derivate der allgemeinen Formel I

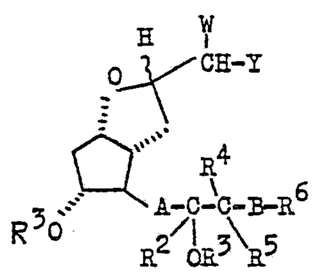

(I)

worin
W —$COOR^1$, —CN, —$NO_2$, $C_{1-4}$-Alkanoyl, $C_{1-4}$-Alkyl-S—, Phenyl-S—, $C_{1-4}$-Alkyl-phenyl-S—, $C_{1-4}$-Alkyl-$SO_2$—, Phenyl-$SO_2$— oder $C_{1-4}$-Alkyl-phenyl-$SO_2$—,
$R^1$ Wasserstoff, $C_{1-6}$-Alkyl, $C_{3-10}$-Cycloalkyl, Benzyl oder Phenyl,
Y Wasserstoff (wenn W $NO_2$ ist), —$COOR^1$, —$COR^1$, —CN oder $-\underset{\underset{Z}{\|}}{C}-X-Q$ bedeutet, worin
Z zwei Wasserstoff (wenn W $NO_2$ ist) oder Sauerstoff,
X —$CH_2$—$CH_2$—, —CH = CH—, —$CH_2$—$C(CH_3)_2$—, —$(CH_3)_2C$—$CH_2$—, Phenyl-S—CH—$CH_2$—, —$CH_2$—CH—S-Phenyl oder Cyclopropylen,
Q —COOH, —$COOR^1$, —$CH_2OH$, —$COL^1$,
M ein pharmakologisch verträgliches Kation und
$L^1$ —$NH_2$, —$NHR^1$ oder —$NR^1R^1$ ist,
A trans-CH = CH—, —C ≡ C— oder —$CH_2$—$CH_2$—,
$R^2$ Wasserstoff der α- oder β-Konfiguration, Methyl oder Äthyl,
$R^3$ Wasserstoff, $C_{1-4}$-Alkanoyl, gegebenenfalls substituiertes Aroyl- oder eine Tri-$C_{1-4}$-Alkyl-silyl-Schutzgruppe,
$R^4$ und $R^5$ unabhängig voneinander Wasserstoff oder $C_{1-4}$-Alkyl,
$R^6$ gerades oder verzweigtes $C_{1-6}$-Alkyl, Phenyl, das durch $C_{1-4}$-Alkyl, Halogen oder durch Halogen substituiertes $C_{1-4}$-Alkyl, substituiert sein kann, oder substituiertes Heteroaryl und
B Methylen, Sauerstoff oder —NH bedeuten,
sowie deren mit pharmakologisch verträglichen Kationen gebildeten Salze.

2. 3α,β-(1'-Äthoxy-carbonyl-1'-cyano-methyl)-6β-(3S-hydroxy-oct-1E-enyl)-7α-hydroxy-2-oxabicyclo-[3,3,0] octan.

3. 5-Nitro-$PGI_1$-methylester.
4. Dinatriumsalz von 5-Nitro-$PGI_1$.
5. 5-Nitro-13,14-didehydro-$PGI_1$.
6. Natriumsalz von 5-Nitro-13,14-didehydro-$PGI_1$.
7. Verfahren zur Herstellung von optisch aktiven und racemischen 5-substituierten $PGI_1$-Derivaten der allgemeinen Formel I

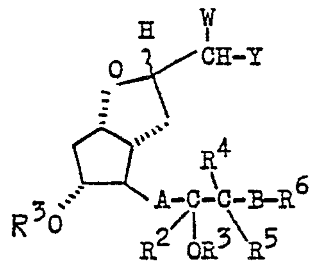

(I)

sowie deren mit pharmakologisch verträglichen Kationen gebildeten Salzen, worin W, $R^1$, Y, $R^2$, $R^3$, $R^4$, $R^5$, $R^6$, A und B die weiter oben angegebene Bedeutung haben, dadurch gekennzeichnet, daß man bicyclische Hemiacetal-Derivate der allgemeinen Formel II

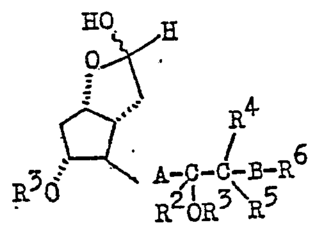

(II)

worin $R^2$, $R^3$, $R^4$, $R^5$, $R^6$, A und B die oben angegebene Bedeutung haben, in Gegenwart eines Katalysators in An- oder Abwesenheit eines Lösungsmitels mit eine aktive Methylengruppe enthaltenden Verbindungen der allgemeinen Formel III

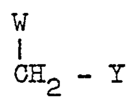

(III)

worin W und Y die oben angegebene Bedeutung haben, umsetzt und in den erhaltenen Verbindungen der allgemeinen Formel I, gewünschtenfalls durch Hydrolyse, Verseifung, Salzbildung, Verersterung, die Einführung einer Schutzgruppe die vorhandenen Substituenten in andere Substituenten der Verbindungen der allgemeinen Formel I überführt.

8. Verfahren nach Anspruch 7, dadurch gekennzeichnet, daß man die Umsetzung in Gegenwart von im Ueberschuß angewandter Verbindung der allgemeinen Formel III oder von aromatischen Kohlenwasserstoffen, vorteilhaft Benzol, Toluol oder Xylol, aliphatischen oder aromatischen halogenierten Kohlenwasserstoffen, vorteilhaft Dichloräthan oder Chlorbenzol, oder dipolaren aprotischen Lösungsmitteln, vorteilhaft Dimethylsulfoxyd, Dimethylformamid oder Hexamethyl-phosphorsäuretriamid durchführt.

9. Verfahren nach Anspruch 7 oder 8, dadurch gekennzeichnet, daß man als Katalysator organische Amine, deren Salze, organische Säuren, Sulfonsäuren oder Lewis-Säuren anwendet.

10. Verfahren nach Anspruch 7, 8 oder 9, dadurch gekennzeichnet, daß man als Katalysator Piperidinium-acetat, Piperidin, eine Lewis-Säure, vorteilhaft Bortrifluorid-ätherat, Zink [II]-chlorid oder Titan [IV]-chlorid verwendet.

11. Pharmazeutische Präparate, die als Wirkstoff eine oder mehrere 5-substituierte $PGI_1$-Derivate der Ansprüche 1-6 enthalten.

**Claims**

1. Optically active and racemic 5-substituted $PGI_1$ derivatives of general formula I

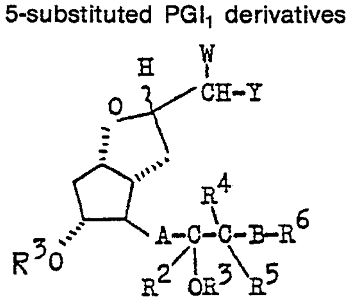

(I)

wherein

W represents $-COOR^1$, $-CN$, $-NO_2$, $C_{1-4}$ alkanoyl, $C_{1-4}$alkyl-S—, phenyl-S—, $C_{1-4}$-alkyl-phenyl-S—. $C_{1-4}$-alkyl-$SO_2$—, phenyl-$SO_2$— or $C_{1-4}$-alkyl-phenyl-$SO_2$—,

$R^1$ represents hydrogen, $C_{1-6}$ alkyl, $C_{3-10}$ cycloalkyl, benzyl or phenyl,

Y represents hydrogen (if W is $NO_2$), $-COOR^1$, $-COR^1$, $-CN$ or $-\underset{\underset{Z}{\|}}{C}-X-Q$, wherein

Z represents two hydrogens (if W is $NO_2$) or oxygen,

X represents $-CH_2-CH_2-$, $-CH=CH-$, $-CH_2-C(CH_3)_2-$, $-(CH_3)_2C-CH_2-$, phenyl-S—CH—$CH_2$, $-CH_2-$CH—S-phenyl or cyclopropylene,

Q represents $-COOH$, $-COOR^1$, $-CH_2OH$, $-COL^1$,

M represents a pharmacologically acceptable cation and

$L^1$ represents $-NH_2$, $-NHR^1$ or $-NR^1R^1$,

A represents trans-$CH=CH-$, $-C\equiv C-$ or $-CH_2-CH_2-$,

$R^2$ represents hydrogen in the α or β configuration or methyl or ethyl,

$R^3$ represents hydrogen, $C_{1-4}$ alkanoyl, optionally substituted aroyl or a tri-$C_{1-4}$ alkyl-silyl protecting group,

$R^4$ and $R^5$ independently of each other represent hydrogen or $C_{1-4}$ alkyl,

$R^6$ represents straight-chained or branched $C_{1-6}$ alkyl, phenyl (which may be substituted by $C_{1-4}$ alkyl, halogen or halogen-substituted $C_{1-4}$ alkyl) or substituted heteroaryl, and

B represents methylene, oxygen or —NH,

and the salts thereof formed with pharmacologically acceptable cations.

2. 3α,β-(1′-Ethoxy-carbonyl-1′-cyano-methyl)-6β-(3S-hydroxy-oct-1E-enyl)-7α-hydroxy-2-oxabicyclo-[3,3,0] octane.

3. 5-Nitro-$PGI_1$ methyl ester.

4. Disodium salt of 5-nitro-$PGI_1$.

5. 5-Nitro-13,14-didehydro-$PGI_1$.

6. Sodium salt of 5-nitro-13,14-didehydro-$PGI_1$.

7. Process for preparing optically active and racemic 5-substituted $PGI_1$ derivatives of general formula I

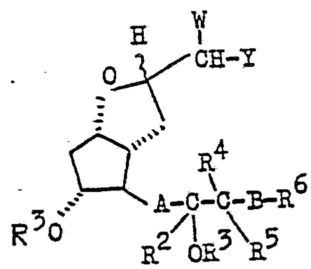

(I)

and the salts thereof formed with pharmacologically acceptable cations, wherein W, $R^1$, Y, $R^2$, $R^3$, $R^4$, $R^5$, $R^6$, A and B have the meaning given hereinbefore, characterised in that bicyclic hemiacetal derivatives of general formula II

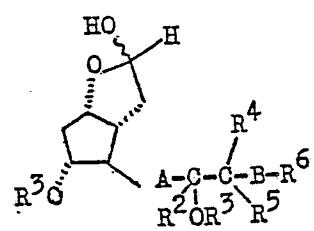

(II)

wherein $R^2$, $R^3$, $R^4$, $R^5$, $R^6$, A and B are as hereinbefore defined, are reacted in the presence of a catalyst in the presence or absence of a solvent with compounds of general formula III

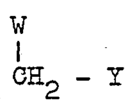

(III)

(wherein W and Y are as hereinbefore defined) which contain an active methylene group, and in the resulting compounds of general formula I the substituents present are converted into other substituents of the compounds of general formula I, if desired by hydrolysis, saponification, salt formation, esterification or the introduction of a protecting group.

8. Process as claimed in claim 7, characterised in that the reaction is carried out in the presence of a compound of general formula III used in excess or in the presence of aromatic hydrocarbons,

advantageously benzene, toluene, or xylene, aliphatic or aromatic halogenated hydrocarbons, advantageously dichlorethane or chlorobenzene, or dipolar aprotic solvents, advantageously dimethylsulphoxide, dimethylformamide or hexamethyl-phosphoric acid triamide.

9. Process as claimed in claim 7 or 8, characterised in that organic amines, the salts thereof, organic acids, sulphonic acids or Lewis acids are used as catalyst.

10. Process as claimed in claim 7, 8 or 9, characterised in that piperidinium acetate, piperidine, a Lewis acid, advantageously boron trifluoride etherate, zinc [II] chloride or titanium [IV] chloride are used as catalyst.

11. Pharmaceutical preparations which contain, as active substance, one or more 5-substituted $PGI_1$ derivatives according to claims 1-6.

**Revendications**

1. Dérivés de $PGI_1$-5-substitués optiquement actifs et racémiques de formule générale I

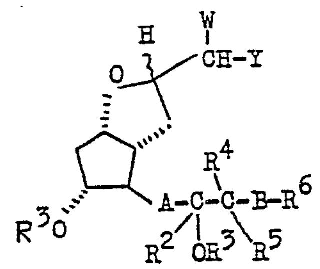

(I)

dans laquelle

W représente $—COOR^1$, —CN, $—NO_2$, un alcanoyle en $C_1$ à $C_4$, un alkyl $C_1$ à $C_4$-S—, un phényl-S—, un alkyl $C_1$ à $C_4$ phényl-S—, un alkyl $C_1$ à $C_4$-$SO_2$—, un phényl-$SO_2$— ou un alkyl $C_1$ à $C_4$-phényl-$SO_2$—,

$R^1$ l'hydrogène, un alkyle en $C_1$ à $C_6$, un cycloalkyle en $C_3$ à $C_{10}$, un benzyle ou un phényle,

Y l'hydrogène (lorsque W est $NO_2$), $—COOR^1$, $—COR^1$, —CN ou $—\underset{\underset{Z}{\|}}{C}—X—Q$ où

Z représente deux hydrogènes (lorsque W est $NO_2$) ou l'oxygène,

X est $—CH_2—CH_2—$, —CH = CH—, $—CH_2—C(CH_3)_2—$, $—(CH_3)_2C—CH_2—$, un phényl-S—CH—$CH_2$—, un $—CH_2—CH—$S-phényle ou un cyclopropylène,

Q est —COOH, $—COOR^1$, $—CH_2OH$, $—COL^1$,

M est un cation pharmacologiquement acceptable et

$L^1$ est $—NH_2$, $—NHR^1$ ou $—NR^1R^1$,

A représente un —CH = CH-trans, —C ≡ C— ou $—CH_2—CH_2—$,

$R_2$ l'hydrogène de configuration α ou β, un méthyle ou un éthyle,

$R_3$ l'hydrogène, un alcanoyle en $C_1$ à $C_4$, un aroyle éventuellement substitué ou un groupe protecteur tri-alkyl $C_1$ à $C_4$-silyle,

$R_4$ et $R_5$ représentent, indépendamment l'un de l'autre, l'hydrogène ou un alkyle en $C_1$ à $C_4$,

$R_6$ représente un alkyle en $C_1$ à $C_6$ droit ou ramifié, un phényle pouvant être substitué par au moins un substituant dans le groupe comportant alkyle en $C_1$ à $C_4$, halogène et alkyle en $C_1$ à $C_4$ substitué par au moins un halogène, ou un hétéroaryle substitué, et

B un méthylène, l'oxygène ou —NH,

ainsi que leurs sels formés avec des cations pharmacologiquement compatibles.

2. 3 α,β-(l'-éthoxy-carbonyl-1'-cyanométhyl-6β)-(3S-hydroxy-oct-1E-ényl)-7α-hydroxy-2-oxabicyclo [3,3,0] octane.

3. Ester méthylique de 5-nitro-$PGI_1$.

4. Sel disodique de 5-nitro-$PGI_1$.

5. 5-nitro-13,14-didéhydro-$PGI_1$.

6. Sel de sodium de 5-nitro-13,14-didéhydro-$PGI_1$.

7. Procédé pour la préparation de dérivés de $PGI_1$ 5-substitués optiquement actifs et racémiques de formule générale I

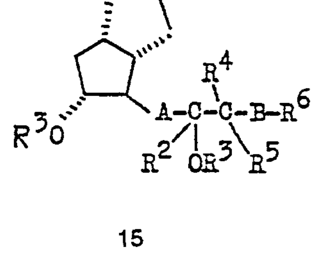

(I)

ainsi que de leurs sels formés avec des cations pharmacologiquement acceptables, formule dans laquelle W, $R^1$, Y, $R^2$, $R^3$, $R^4$, $R^5$, $R^6$, A et B ont la signification indiquée précédemment, caractérisé en ce que l'on fait réagir des dérivés hémiacétals bicycliques de formule générale II

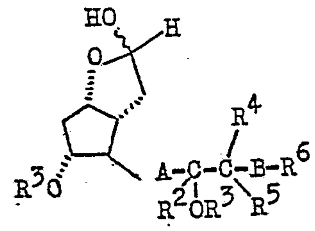

(II)

dans laquelle $R^2$, $R^3$, $R^4$, $R^5$, $R^6$, A et B ont la signification indiquée précédemment, en présence d'un catalyseur avec ou sans solvant, avec des composés renfermant un groupe méthylène actif de formule générale III

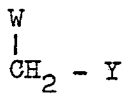

(III)

où W et Y ont la signification indiquée plus haut et que l'on convertit dans les composés de formule générale I obtenus, le cas échéant, par hydrolyse, saponification, salification, estérification, introduction d'un groupe protecteur, les substituants présents en d'autres substituants des composés de formule générale I.

8. Procédé selon la revendication 7, caractérisé en ce qu'on effectue la réaction en présence du composé de formule générale III utilisé en excès ou en présence d'hydrocarbures aromatiques, avantageusement le benzène, le toluène ou le xylène, d'hydrocarbures aliphatiques ou aromatiques halogénés, avantageusement le dichloréthane ou le chlorobenzène ou de solvants aprotiques dipolaires, avantageusement le diméthylsulfoxyde, le diméthylformamide ou l'hexaméthylphosphorotriamide.

9. Procédé selon la revendication 7 ou 8, caractérisé en ce qu'on utilise comme catalyseur des amines organiques, leurs sels, des acides organiques, des acides sulfoniques ou des acides de Lewis.

10. Procédé selon la revendication 7, 8, ou 9, caractérisé en ce qu'on utilise comme catalyseur l'acétate de pipéridinium, la pipéridine, un acide de Lewis, avantageusement le trifluorure de bore-éther, le chlorure de zinc (II) ou le chlorure de titane (IV).

11. Préparations pharmaceutiques renfermant, à titre de principe actif, un ou plusieurs dérivés de $PGI_1$ 5-substitués des revendications 1 à 6.